(19) Europäisches Patentamt
European Patent Office
Office européen des brevets

(11) Publication number: 0 136 729 B1

(12) # EUROPEAN PATENT SPECIFICATION

(45) Date of publication of patent specification: 03.02.88

(51) Int. Cl.⁴: C 07 D 213/64, C 07 D 213/63, C 07 C 145/04, A 01 N 47/34

(21) Application number: 84200967.2

(22) Date of filing: 03.07.84

(54) **Pesticidal benzoylurea derivatives.**

(30) Priority: 26.07.83 GB 8320155

(43) Date of publication of application: 10.04.85 Bulletin 85/15

(45) Publication of the grant of the patent: 03.02.88 Bulletin 88/05

(84) Designated Contracting States: AT BE CH DE FR GB IT LI LU NL SE

(56) References cited:
EP-A-0 025 363
EP-A-0 103 918
GB-A-2 118 941

(73) Proprietor: SHELL INTERNATIONALE RESEARCH MAATSCHAPPIJ B.V.
Carel van Bylandtlaan 30
NL-2596 HR Den Haag (NL)

(72) Inventor: Anderson, Martin
92 Clare Road
Whitstable Kent CT5 2EH (GB)

(74) Representative: Hunter, Keith Roger Ian et al
4 York Road
London SE1 7NA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 136 729 B1

Courier Press, Leamington Spa, England.

**Description**

The present invention relates to benzoylurea compounds and their preparation and to compositions containing such derivatives and their use as pesticides.

Certain benzoylurea compounds are known as insecticides, for example see GB 1460 410 and GB 1501 607. It has now been found that certain novel benzoylurea derivatives also have useful pesticidal properties.

Accordingly the present invention provides a benzoylurea derivative of the general formula I,

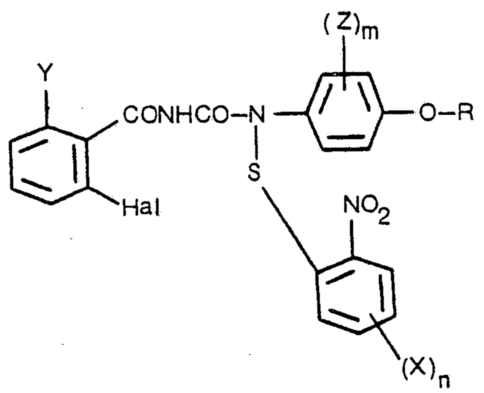

(I)

wherein R represents a pyridyl group optionally substituted by one or more of the same or different substituents selected from the group consisting of halogen, cyano, alkyl, nitro, haloalkyl and alkoxy; or an alkenyl group optionally substituted by one or more halogen atoms; each of X and Z independently represents a halogen atom or an alkyl, haloalkyl, cyano, alkoxy or nitro group; Y represents a hydrogen or halogen atom; Hal represents a halogen atom; and each of n and m is 0, 1, 2 or 3.

Throughout this specification, unless otherwise stated, any alkyl, alkoxy or alkenyl group present contains up to 6, especially up to 4, carbon atoms.

When the pyridyl group R is substituted by a haloalkyl group or when X and/or Z represents a haloalkyl group, a preferred example of such a haloalkyl group is a trihaloalkyl group, particularly trifluoromethyl. Preferably the group R is a pyridyl group optionally substituted by one or more of the same or different substituents selected from halogen atoms and haloalkyl groups or R is an alkenyl group.

Suitable examples of alkenyl groups include 2-propenyl (allyl), 2-butenyl, and 3-butenyl.

When R is a pyridyl group this is preferably a 2-pyridyl group, particularly one optionally substituted by chlorine atoms. Most preferably R is an unsubstituted or dichloro-substituted pyridyl group, the substituents situated in the 3- and 5-positions; an alkenyl group R is preferably a 2-propenyl (allyl) group.

Y is preferably a halogen atom. The halogen atom represented by Y or by Hal may be fluorine, chlorine or bromine but it is preferably fluorine or chlorine. Most preferred are those compounds of formula I wherein both Y and Hal represent fluorine atoms.

Each of the X substituents may be located in any one of the 4 remaining positions on the sulphenyl ring in formula I. Most preferably these 4 positions are unsubstituted and n is O.

Preferably each Z independently represents a halogen atom. Suitable atoms are the same as those listed above for Y and Hal. Most preferably each Z independently represents chlorine or fluorine. Especially preferred are those compounds of formula I wherein m is O, 1 or 2. When m is 1 or 2 the or each Z is preferably chlorine, situated on the phenyl ring in the 3-position (m=1) or 3,5-positions (m=2) relative to the nitrogen atom.

Depending on the various groups present in the molecule, the compounds of the general formula I may exist in the form of geometric and/or optical isomers. The invention should be understood to include all individual isomers and mixtures thereof.

The invention also provides a process for preparing the novel benzoylurea derivatives which comprises reacting a benzoylisocyanate of formula:

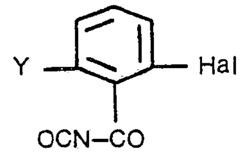

(II)

with a sulphenamide of formula:

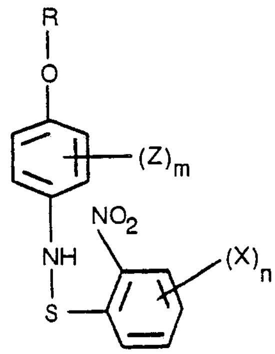

(III)

wherein Y, Hal, R, X, Z, n and m have the meanings specified above. Preferably the reaction is carried out under anhydrous conditions. It is also preferred that the reaction is carried out in the presence of a solvent.

Suitable solvents are aromatic solvents such as benzene, toluene, xylene, or chlorobenzene, hydrocarbons such as petroleum (40°—60°C), chlorinated hydrocarbons such as chloroform, methylene chloride or ethylene chloride, and ethers such as diethylether, dibutylether, or dioxan. Mixtures of solvents are often suitable.

Preferably the reaction is carried out at a temperature of 0 to 100°C, suitable ambient temperature. Preferably the ratio of benzoylisocyanate to sulphenamide is from 1:1 to 2:1. The product may be worked up by usual techniques. The starting materials of formula II are known compounds and may be prepared by methods analogous to those known in the art.

The starting materials of formula III may be prepared by reacting a sulphenyl halide of formula:

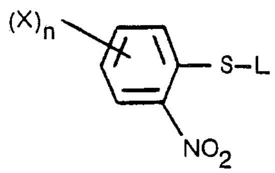

(IV)

with an aniline of formula:

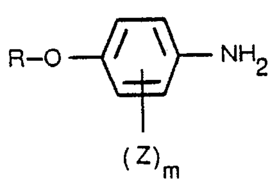

(V)

wherein R, X, Z, n and m have the meanings specified above in relation to formula I and L represents a halogen atom, preferably a chlorine or bromine atom. The reaction is preferably carried out in the presence of a dehydrohalogenating agent, such as an organic or inorganic base. Suitable bases include alkali metal hydroxides, amides or alkoxides, for example, sodium ethoxide; alkali metal or alkaline earth metal hydroxides, for example, potassium hydroxide; and primary, secondary or tertiary amines, for example, triethylamine or piperidine.

The reaction is conveniently carried out in the presence of an aprotic solvent; suitable solvents are aromatic solvents such as benzene, toluene, xylene or chlorobenzene, hydrocarbons such as petroleum, chlorinated hydrocarbons, such as methylene chloride, and ethers such as diethylether and dibutylether.

The reaction is suitably carried out at a temperature in the range of −10° to 50°C, preferably −5° to 20°C.

The sulphenyl halides of formula IV may be prepared from the corresponding thiol or disulphide, by reaction with the appropriate halogen by methods analogous to those known in the art. In the preparation of the intermediates III the sulphenyl halide IV starting material may be prepared *in situ*.

The compounds of the present invention have been found to have high insecticidal and acaricidal activity. Accordingly the present invention also provides pesticidal compositions comprising a compound of formula I as defined above together with a carrier. Such a composition may contaion a single compound or a mixture of several compounds of the invention. The invention further provides a method of combating pests at a locus, which comprises applying an insecticidally effective amount of a compound or composition according to the present invention to the locus. The dosage at which the active compound is applied will depend upon the locus to be treated. Typical dose rates for agricultural outlets are, for example, 0.005kg/ha to 2kg/ha, preferably 0.010kg/ha to 1kg/ha; for public health outlets dose rates are, for

example, 0.005ppm to 2,000ppm, especially 0.01ppm to 1,000ppm. Unlike the compounds of the prior art, which are virtually insoluble in organic solvents and therefore extremely difficult to formulate without great expense, the compounds of the general formula I are in general more soluble in organic solvents making formulation relatively simple, and the resulting compositions easy to handle and economically attractive.

A composition according to the invention comprises one or more carriers. A carrier is any material with which the active ingredient are formulated to facilitate application to the locus to be treated, or to facilitate storage, transport or handling, and any of the carriers normally used in formulating pesticidal compositions may be used. Preferably compositions according to the invention contain 0.5 to 95% by weight of active ingredient. Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent, for example, the composition may contain at least two carriers, at least one of which is a surface-active agent. Examples of carriers which may be used in compositions according to the invention may be found for example in GB 2112644 A.

The compositions of the invention may also contain other ingredients, for example, other compounds possessing pesticidal, herbicidal or fungicidal properties. Further insecticidal compounds may be included, particularly such compounds having a different mode of activity, such as more rapid knock down than the compounds of the present invention. Examples are pyrethrum or pyrethroid compounds such as permethrin, cypermethrin, deltamethrin and fenvalerate.

The following Examples illustrate the invention. Examples 1 and 2 illustrate the preparation of sulphenamide intermediates of formula III.

## Example 1

Preparation of N-[3-chloro-4[(3,5-dichloro-2-pyridinyl)oxy]phenyl]-2-nitrobenzenesulphenamide

A solution of 2-nitrobenzenesulphenyl chloride (5.3g) in dry dichloromethane (75mls) was added to a stirred solution of 3-chloro-4[(3,5-dichloro-2-pyridinyl)oxy]aniline (7.24g) and triethylamine (2.93g) in the same solvent (30mls) over 10 minutes. After stirring for 5 hours at room temperature the solvent was evaporated, and the residual red oil dissolved in diethylether. The solution was then washed twice with water, dried in solution over sodium sulphate and the solvent evaporated to leave a resin. The resin was redissolved in dichloromethane, evaporated to dryness and left at a pressure of 0.1mm Hg overnight. The product was recrystallised from petroleum ether (40°—60°C) to yield 10.8g (corresponding to 97.6%) of bright yellow resin, mpt 146—147°C; the following elemental analysis results were obtained:

| | | | |
|---|---|---|---|
| Calculated for $N_3SO_3Cl_3C_{17}H_{10}$: | C:46.1 | H:2.3 | N:9.5 |
| Found: | C:48.2 | H:2.6 | N:8.7 |

## Example 2

Preparation of N-[3,5-dichloro-4-(2-propenyloxy)phenyl]-2-nitrobenzenesulphonamide

A solution of 3,5-dichloro-4-(2-propenyloxy)aniline (4g) in dry dichloromethane (30mls) was stirred at ambient temperature and treated firstly with triethylamine (2.11g) and then with a solution of 2-nitrobenzenesulphenylchloride (3.83g) in dichloromethane. The mixture was stirred overnight at room temperature, the solvent evaporated and the residual brown gum diluted with water (100mls).

The solution was extracted twice with diethylether (2x100mls), the extracts washed with saturated sodium chloride, dried over sodium sulphate and evaporated to leave a brown resin. The resin was redissolved in dichloromethane and chromatographed on silica using dichloromethane as an eluent.

The product was recrystallised from dichloromethane/petroleum ether (60°—80°C) to give 5.9g (corresponding to a yield of 67.9%) of yellow, crystalline solid; mpt 138—139°C. The following elemental analysis results were obtained:

| | | | |
|---|---|---|---|
| Calculated for $N_2SO_3Cl_2C_{15}H_{12}$ | C:48.5 | H:3.3 | N:7.5 |
| Found: | C:48.4 | H:3.2 | N:7.4 |

Examples 3 to 5 illustrate the preparation of benzoylurea derivatives of the formula I.

## Example 3

Preparation of N-[[[3-chloro-4-[(3,5-dichloro-2-pyridinyl)oxy]phenyl][(2-nitrophenyl)thio]amino]carbonyl]-2,6-difluorobenzamide

A solution of 2,6-difluorobenzoylisocyanate (4.03g) in dry toluene (20mls) was added over 10 minutes to a stirred solution of the compound of example 1 (8.85g) in dry toluene (85mls) at 40°C. The heat was removed and the orange-coloured solution stirred at ambient temperature overnight. The solution was then cooled to −5°C, filtered, washed firstly with cold toluene (at 0°C) and then washed with petroleum ether (40°—60°C) and dried over vacuum at ambient temperature to yield 8.3g (corresponding to 66.3%) of product; mpt 185°C; the following elemental analysis results were obtained:

| | | | |
|---|---|---|---|
| Calculated for $N_4SO_5Cl_3F_2C_{25}H_{13}$: | C:48.0 | H:2.1 | N:9.0 |
| Found: | C:48.2 | H:1.8 | N:8.6 |

## Example 4

Preparation of N-[[[3,5-dichloro-4-(2-propenyloxy)phenyl][(2-nitrophenyl)thio]amino]carbonyl]-2,6-difluorobenzamide

A solution of 2,6-difluorobenzoylisocyanate (1.62g) in dry toluene (10mls) was added over about 5 minutes to a solution of the compound of Example 2 (3.0g) in dry toluene (10mls). The resulting solution was stirred for 2 days at room temperature, then cooled to about 0°C, filtered and the precipitate washed with petroleum ether (40°—60°C). The product was dried under vacuum at 70°C; mpt 171—173°C; yield 3.7gms (corresponding to 82.6%). The following elemental analysis results were obtained:

| | | | |
|---|---|---|---|
| Calculated for $N_3SO_5Cl_2F_2C_{23}H_{15}$ | C:49.8 | H:2.7 | N:7.6 |
| Found | C:50.1 | H:2.7 | N:7.6 |

## Example 5

Preparation of 2,6-dichloro-N-[[[3-chloro-4-[(3,5-dichloro-2-pyridinyl)oxy]phenyl][(2-nitrophenyl)thio]amino]carbonyl]benzamide.

By a method analogous to that described in Example 3, this compound was prepared from the compound of Example 1. mpt 178—180°C; the following elemental analysis results were obtained:

| | | | |
|---|---|---|---|
| Calculated for $N_4SO_5Cl_5C_{25}H_{13}$ | C:45.6 | H:2.0 | N:8.5 |
| Found: | C:45.8 | H:1.9 | N:9.1 |

## Example 6

The insecticidal activity of the compounds of the invention was determined in the following tests.

Test 1

The insecticidal and ovicidal activities of the compounds of the invention were assessed employing the pests *Spodoptera littoralis* (S.1.) and *Aedes aegypti* (A.a)

The test methods used for each species appear below. In each case the tests were conducted under normal conditions (23°C ± 2°C; fluctuating light and humidity).

In each test an $LC_{50}$ for the compound was calculated from the mortality figures and compared with the corresponding $LC_{50}$ for ethyl parathion in the same tests. The results are expressed as toxicity indices =

$$\frac{LC_{50}\text{ (parathion)}}{LC_{50}\text{ (test compound)}} \times 100$$

and are set out in Table 1 below.

(i) *Spodoptera Littoralis*

Solutions or suspensions of the compound were made up over a range of concentrations in 10% acetone/water containing 0.025% Triton X100 ("Triton" is a registered trade mark). These solutions were sprayed using a logarithmic spraying machine onto petri dishes containing a nutritious diet on which the *Spodoptera littoralis* larvae had been reared. When the spray deposit had dried each dish was infested with 10 2nd instar larvae. Mortality assessments were made 7 days after spraying.

(ii) *Aedes aegypti*

Several solutions of the test compound of varying concentration were prepared in acetone. 100 microlitre quantities were added to 100ml of tap water, the acetone being allowed to evaporate off. 10 early 4th instar larvae were placed in the test solution; after 48 hours the (surviving) larvae were fed with animal feed pellets, and the final percentage mortality assessed when all the larvae had either pupated and emerged as adults or died.

TABLE 1

| Compound of Example | S.1. | Test code A.a. |
|---|---|---|
| 3 | 1100 | 530 |
| 4 | 170 | 15 |
| 5 | 170 | 3 |

**Claims for the Contracting States: BE CH DE FR GB IT LI LU NL SE**

1. A benzoylurea derivative of the general formula I

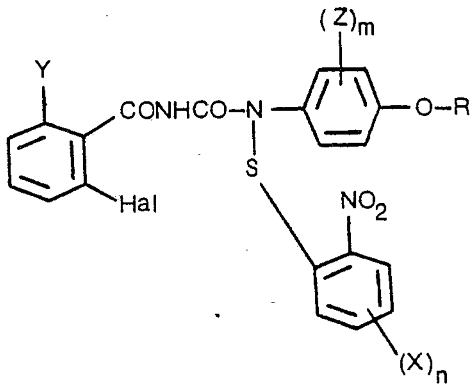

(I)

wherein R represents a pyridyl group optionally substituted by one or more of the same or different substituents selected from the group consisting of halogen, cyano, alkyl, nitro, haloalkyl and alkoxy; or an alkenyl group optionally substituted by one or more halogen atoms; each of X and Z independently represents a halogen atom or an alkyl, haloalkyl, cyano, alkoxy or nitro group; Y represents a hydrogen or halogen atom; Hal represents a halogen atom; and each of n and m is independently 0, 1, 2 or 3; any alkyl, alkoxy or alkenyl group present containing up to 6 carbon atoms.

2. A compound as claimed in claim 1 wherein R represents a pyridyl group optionally substituted by one or more of the same or different substituents selected from halogen atoms and haloalkyl groups, or an alkenyl group.

3. A compound as claimed in claim 1 or claim 2 wherein R represents a 2-pyridyl, a 3,5-dichloro-2-pyridyl or a 2-propenyl group.

4. A compound as claimed in any one of the preceding claims wherein Y represents a halogen atom.

5. A compound as claimed in claim 4 wherein Y and Hal both represent fluorine atoms.

6. A compound as claimed in any one of the preceding claims wherein n is 0.

7. A compound as claimed in any one of the preceding claims wherein m is 0, 1 or 2 and when m is 1, Z represents a 3-chloro substituent and when m is 2, Z represents a 3,5-dichloro substituent.

8. A process for the preparation of a compound as claimed in claim 1 which process comprises reacting a benzoylisocyanate of formula:—

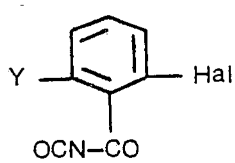

(II)

with a sulphenamide of formula

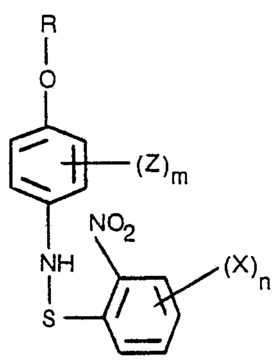

(III)

in which Y, Hal, R, Z, X, n and m have the meanings specified in claim 1.

9. A pesticidal composition comprising a compound as claimed in any one of claims 1 to 7 together with a carrier.

10. A method of combating pests at a locus which comprises applying a pesticidally effective amount of a compound according to any one of claims 1 to 7 or a composition according to claim 9 to the locus.

**Claims for the Contracting State: AT**

1. A process for the preparation of a benzoylurea derivative of the general formula I

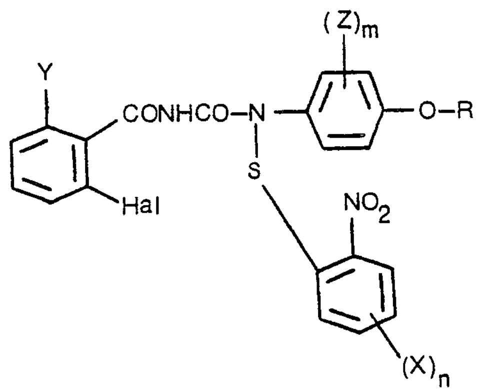

(I)

wherein R represents a pyridyl group optionally substituted by one or more of the same or different substituents selected from the group consisting of halogen, cyano, alkyl, nitro, haloalkyl and alkoxy; or an alkenyl group optionally substituted by one or more halogen atoms; each of X and Z independently represents a halogen atom or an alkyl, haloalkyl, cyano, alkoxy or nitro group; Y represents a hydrogen or halogen atom; Hal represents a halogen atom; and each of n and m is independently 0, 1, 2 or 3; any alkyl, alkoxy or alkenyl group present containing up to 6 carbon atoms, which comprises reacting a benzoylisocyanate of formula:—

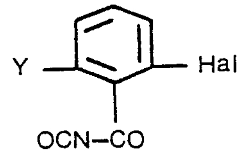

(II)

with a sulphenamide of formula

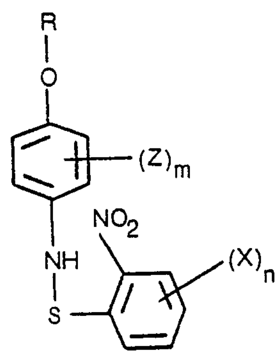

(III)

in which Y, Hal, R, Z, X, n and m have the meanings given above.

2. A process as claimed in claim 1 wherein R represents a pyridyl group optionally substituted by one or more of the same or different substituents selected from halogen atoms and haloalkyl groups, or an alkenyl group.

3. A process as claimed in claim 1 or claim 2 wherein R represents a 2-pyridyl, a 3,5-dichloro-2-pyridyl or a 2-propenyl group.

4. A process as claimed in any one of the preceding claims wherein Y represents a halogen atom.

5. A process as claimed in claim 4 wherein Y and Hal both represent fluorine atoms.

6. A process as claimed in any one of the preceding claims wherein n is 0.

7. A process as claimed in any one of the preceding claims wherein m is 0, 1 or 2 and when m is 1, Z represents a 3-chloro substituent and when m is 2, Z represents a 3,5-dichloro substituent.

8. A pesticidal composition comprising a compound of formula I as defined in any one of claims 1 to 7 together with a carrier.

9. A composition as claimed in claim 8, which comprises at least two carriers, at least one of which is a surface active agent.

10. A method of combating pests at a locus which comprises applying a pesticidally effective amount

of a compound of formula I as defined in any one of claims 1 to 7 or a composition as claimed in either claim 8 or claim 9 to the locus.

**Patentansprüche für die Vertragstaaten: BE CH DE FR GB IT LI LU NL SE**

1. Benzoylharnstoffderivat der allgemeinen Formel I:

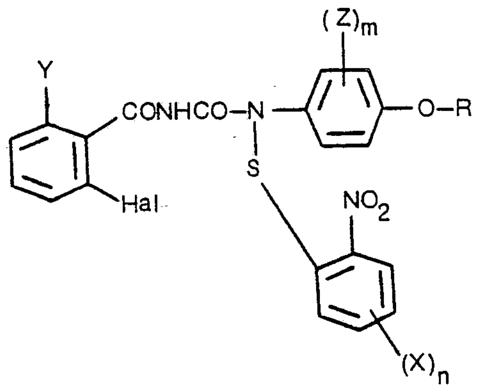

(I)

worin R eine Pyridylgruppe darstellt, die gegebenenfalls durch einen oder mehrere, gleiche oder verschiedene Substituenten, ausgewählt aus der aus Halogen, Cyano, Alkyl, Nitro, Halogenalkyl und Alkoxy bestehenden Gruppe, substituiert ist; oder eine Alkenylgruppe darstellt, die gegebenenfalls durch ein oder mehrere Halogenatome substituiert ist; jeder der Reste X und Z unabhängig voneinander ein Halogenatom oder eine Alkyl-, Halogenalkyl-, Cyano-, Alkoxy- oder Nitrogruppe bedeutet; Y ein Wasserstoffatom oder ein Halogenatom darstellt; Hal ein Halogenatom bedeutet; und n und m jeweils unabhängig voneinander für 0, 1, 2 oder 3 stehen; wobei etwa vorliegende Alkyl-, Alkoxy- oder Alkenylgruppen bis zu 6 Kohlenstoffatome aufweisen.

2. Verbindung nach Anspruch 1, worin R eine Pyridylgruppe darstellt, die gegebenenfalls durch einen oder mehrere, gleiche oder verschiedene Substituenten, ausgewählt unter Halogenatom und Halogenalkylgruppen, substituiert ist, oder eine Alkenylgruppe bedeutet.

3. Verbindung nach Anspruch 1 oder 2, worin R eine 2-Pyridylgruppe, eine 3,5-Dichlor-2-pyridylgruppe oder eine 2-Propenylgruppe bedeutet.

4. Verbindung nach einem der vorstehenden Ansprüche, worin Y ein Halogenatom darstellt.

5. Verbindung nach Anspruch 4, worin Y und Hal beide Fluoratome bedeuten.

6. Verbindung nach einem der vorstehenden Ansprüche, worin n den Wert 0 hat.

7. Verbindung nach einem der vorstehenden Ansprüche, worin m den Wert 0, 1 oder 2 aufweist, und, wenn m 1 ist, Z einen 3-Chlorsubstituenten bedeutet, und wenn m 2 ist, Z einen 3,5-Dichlorsubstituenten darstellt.

8. Verfahren zur Herstellung einer Verbindung nach Anspruch 1, welches Verfahren ein Umsetzen eines Benzoylisocyanats der Formel:

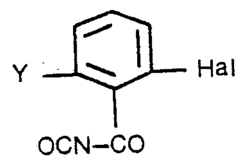

(II)

mit einem Sulfenamid der Formel

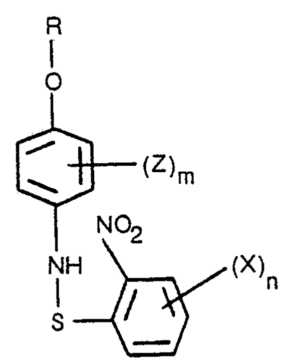

(III)

worin Y, Hal, R, Z, X, n und m die in Anspruch 1 angegebenen Bedeutungen aufweisen, umfaßt.

9. Pestizide Zusammensetzung, enthaltend eine Verbindung nach einem der Ansprüche 1 bis 7, zusammen mit einem Träger.

10. Verfahren zur Bekäm pfung von Schädlingen an einem Ort, welches ein Aufbringen einer pestizid wirksamen Menge einer Verbindung nach einem der Ansprüche 1 bis 7 oder eine Zusammensetzung nach Anspruch 9 auf den Ort umfaßt.

**Patentansprüche für den Vertragsstaat: AT**

1. Verfahren zur Herstellung eines Benzoylharnstoffderivats der allgemeinen Formel I

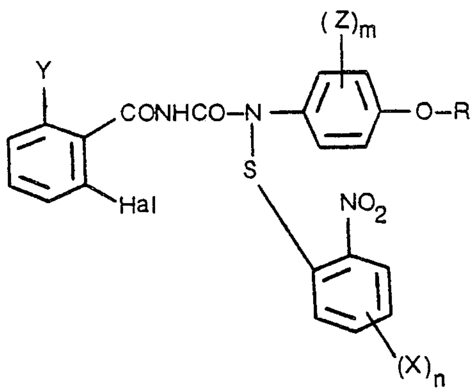

(I)

worin R eine Pyridylgruppe darstellt, die gegebenenfalls durch einen oder mehrere, gleiche oder verschiedene Substituenten, ausgewählt aus der aus Halogen, Cyano, Alkyl, Nitro, Halogenalkyl und Alkoxy bestehenden Gruppe, substituiert ist; oder eine Alkenylgruppe darstellt, die gegebenenfalls durch ein oder mehrere Halogenatome substituiert ist; jeder der Reste X und Z unabhängig voneinander ein Halogenatom oder eine Alkyl-, Halogenalkyl-, Cyano-, Alkoxy- oder Nitrogruppe bedeutet; Y ein Wasserstoffatom oder ein Halogenatom darstellt; Hal ein Halogenatom bedeutet; und n und m jeweils unabhängig voneinander für 0, 1, 2 oder 3 stehen; wobei etwa vorliegende Alkyl-, Alkoxy- oder Alkenylgruppen bis zu 6 Kohlenstoffatome aufweisen, welches Verfahren ein Umsetzen eines Benzoylisocyanats der Formel:

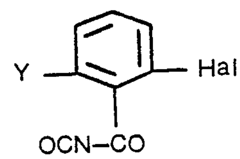

(II)

mit einem Sulfenamid der Formel:

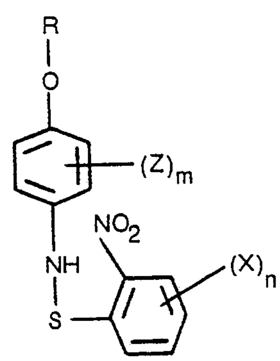

(III)

worin Y, Hal, R, Z, X, n und m die vorstehend angegebenen Bedeutungen aufweisen, umfaßt.

2. Verfahren nach Anspruch 1, worin R eine Pyridylgruppe darstellt, die gegebenenfalls durch einen oder mehrere, gleiche oder verschiedene Substituenten, ausgewählt unter Halogenatomen und Halogenalkylgruppen, substituiert ist, oder eine Alkenylgruppe bedeutet.

3. Verfahren nach Anspruch 1 oder 2, worin R eine 2-Pyridylgruppe, eine 3,5-Dichlor-2-pyridylgruppe oder eine 2-Propenylgruppe bedeutet.

4. Verfahren nach einem der vorstehenden Ansprüche, worin Y ein Halogenatom darstellt.

5. Verfahren nach Anspruch 4, worin Y und Hal beide Fluoratome bedeuten.

6. Verfahren nach einem der vorstehenden Ansprüche, worin n den Wert 0 hat.

7. Verfahren nach einem der vorstehenden Ansprüche, worin m den Wert 0, 1 oder 2 aufweist, und, wenn m 1 ist, Z einen 3-Chlorsubstituenten bedeutet, und wenn m 2 ist, Z einen 3,5-Dichlorsubstituenten darstellt.

8. Pestizide Zusammensetzung, umfassend eine Verbindung der Formel I, wie in einem der Ansprüche 1 bis 7 definiert, zusammen mit einem Träger.

9. Zusammensetzung nach Anspruch 8, welche wenigstens 2 Träger umfaßt, von den wenigstens einer ein oberflächenaktives Mittel ist.

10. Verfahren zur Bekämpfung von Schädlingen an einem Ort, welches ein Aufbringen einer pestizid wirksamen Menge einer Verbindung der Formel I, wie in einem der Ansprüche 1 bis 7 definiert, oder einer Zusammensetzung, wie in Anspruch 8 oder Anspruch 9 beansprucht, auf den Ort umfaßt.

**Revendications pour les Etats contractants: BE CH DE FR GB IT LI LU NL SE**

1. Un dérivé de benzoylurée de la formule générale I

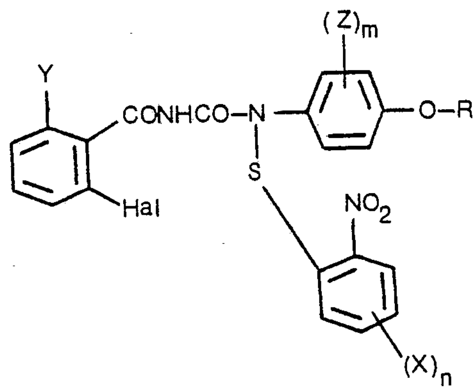

(I)

où R représente un groupe pyridyle éventuellement substitué par un ou plusieurs substituants identiques ou différents choisis dns le groupe constitué par les halogènes et les radicaux cyano, alcoyle, nitro, haloalcoyle et alcoxy; ou un groupe alcényle éventuellement substitué par un ou plusieurs atoms d'halogènes; chacun des X et Z représente indépendamment un atome d'halogène ou un groupe alcoyle, haloalcoyle, cyano, alcoxy ou nitro; Y représente un atome d'hydrogène ou d'halogène; Hal représente un atome d'halogène; et chacun de n et m est indépendamment 0, 1, 2 ou 3; tout groupe alcoyle, alcoxy ou alcényle présent contenant jusqu'à 6 atomes de carbone.

2. Un composé selon la revendication 1, dans lequel R représente un groupe pyridyle éventuellement substitué par un ou plusieurs substituants identiques ou différents choisis parmi des atomes d'halogènes et des groupes haloalcoyle, ou un groupe alcényle.

3. Un composé selon la revendication 1 ou la revendication 2, dans lequel R représente un groupe 2-pyridyle, 3,5-dichloro-2-pyridyle ou 2-propényle.

4. Un composé selon l'une quelconque des revendications précédentes, dans lequel Y représente un atome d'halogène.

5. Un composé selon la revendication 4, dans lequel Y et Hal représentent tous deux des atomes de fluor.

6. Un composé selon l'une quelconque des revendications précédentes, dans lequel n est 0.

7. Un composé selon l'une quelconque des revendications précédentes, dans lequel m est 0, 1 ou 2 et quand m est 1, Z représente un substituant 3-chloro et quand m est 2, Z représente un substituant 3,5-dichloro.

8. Un procédé pour la préparation d'un composé tel que revendiqué dans la revendication 1, procédé qui comprend la réaction d'un benzoylisocyanate de formule:

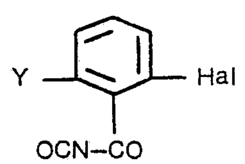

(II)

avec un sulfénamide de formule:

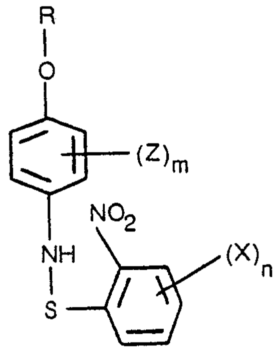

(III)

où Y, Hal, R, Z, X, n et m ont les significations spécifiées dans la revendication 1.

9. Une composition pesticide comprenant un composé selon l'une quelconque des revendications 1 à 7 en même temps qu'un véhicule.

10. Une méthode de lutte contre des organismes nuisibles en un lieu, qui comprend l'application à ce lieu d'une quantité efficace du point de vue pesticide d'un composé selon l'une quelconque des revendications 1 à 7 ou d'une composition selon la revendication 9.

**Revendications pour l'Etat contractant: AT**

1. Un procédé pour la préparation d'un dérivé de benzoylurée de la formule générale I

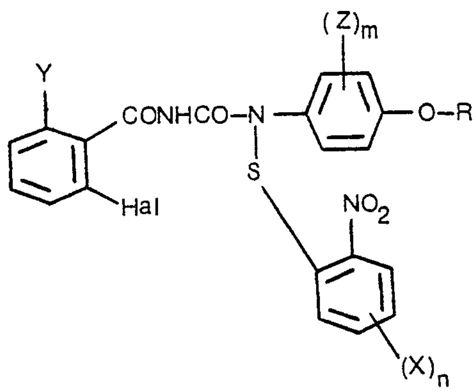

(I)

où R représente un groupe pyridyle éventuellement substitué par un ou plusieurs substituants identiques ou différents choisis dAns le groupe constitué par les halogènes et les radicaux cyano, alcoyle, nitro, haloalcoyle et alcoxy; ou un groupe alcényle éventuellement substitué par un ou plusieurs atoms d'halogènes; chacun des X et Z représente indépendamment un atome d'halogène ou un groupe alcoyle, haloalcoyle, cyano, alcoxy ou nitro; Y représente un atome d'hydrogène ou d'halogène; Hal représente un atome d'halogène; et chacun de n et m est indépendamment 0, 1, 2 ou 3; tout groupe alcoyle, alcoxy ou alcényle préesent contenant jusqu'à 6 atomes de carbone, qui comprend la réaction d'un benzoylisocyanate de formule

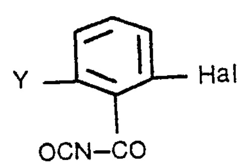

(II)

avec un sulfénamide de formule

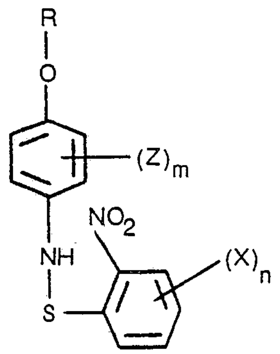

(III)

où Y, Hal, R, Z, X, n et m ont les significations indiquées ci-dessus.

2. Un procédé selon la revendication 1, dans lequel R représente un groupe pyridyle éventuellement substitué par un ou plusieurs substituants identiques ou différents choisis parmi des atomes d'halogènes et des groupes haloalcoyle, ou un groupe alcényle.

3. Un procédé selon la revendication 1 ou la revendication 2, dans lequel R représente un groupe 2-pyridyle, un groupe 3,5-dichloro-2-pyrinyle ou un groupe 2-propényle.

4. Un procédé selon l'une quelconque des revendications précédentes, dans lequel Y représente un atome d'halogène.

5. Un procédé selon la revendication 4, dans lequel Y et Hal représentent tous deux des atomes de fluor.

6. Un procédé selon l'une quelconque des revendications précédentes, dans lequel n est 0.

7. Un procédé selon l'une quelconque des revendications précédentes, dans lequel m est 0, 1 ou 2 et quand m est 1, Z représente un substituant 3-chloro et quand m est 2, Z représente un substituant 3,5-dichloro.

8. Une composition pesticide comprenant un composé de formule I tel que défini dans l'une quelconque des revendications 1 à 7 en même temps qu'un véhicule.

9. Une composition selon la revendication 8, qui comprend au moins deux véhicules, dont l'un au moins est un agent tensio-actif.

10. Une méthode de lutte contre des organismes nuisibles en un lieu, qui comprend l'application à ce lieu d'un composé de formule I tel que défini dans l'une quelconque des revendications 1 à 7 ou d'une composition selon la revendication 7 ou la revendication 8.